# EUROPEAN PATENT APPLICATION

(11) **EP 4 134 453 A1**
(43) Date of publication of application: **15.02.2023**
(21) Application number: 21783723.6
(22) Date of filing: 08.04.2021
(51) Int. Cl.: C12Q 1/6886

(54) **COMPOSITION FOR DIAGNOSING COLORECTAL CANCER, RECTAL CANCER, OR COLORECTAL ADENOMA USING CPG METHYLATION CHANGE OF GLRB GENE, AND USE THEREOF**

(30) Priority: 08.04.2020 KR 20200042726
(71) Applicant: Gencurix Inc., Seoul 08394 (KR)
(72) Inventor: CHO, Sang Rae, Seoul 08394 (KR); MOON, Young Ho, Seoul 08394 (KR); HAN, Jinil, Seoul 08394 (KR); LEE, Yun Young, Seoul 08394 (KR)
(74) Representative: Schwarz & Partner Patentanwälte GmbH
(86) International application number: PCT/KR2021/004411
(87) International publication number: WO 2021/206467

(57) **Abstract**

The present invention relates to a composition, a kit, a nucleic acid chip, and a method capable of diagnosing colorectal cancer, rectal cancer, or colorectal adenoma by detecting the methylation level of CpG sites in a GLRB gene. Accordingly, colorectal cancer, rectal cancer, or colorectal adenoma can be diagnosed not only accurately and quickly, but also at an early stage.

## Description

### TECHNICAL FIELD

This application claims the priority of Korean Patent Application No. 10-2020-0042726, filed on April 8, 2020, the entirety of which is a reference of the present application.

The present invention relates to a composition, a kit, a nucleic acid chip, and a method capable of diagnosing colorectal cancer, rectal cancer, or colorectal adenoma by detecting the methylation level of CpG sites in a GLRB gene.

### BACKGROUND ART

Colon is the last part of the digestive system, has a length of 2 m, and is divided into appendix, ascending colon, transverse colon, descending colon, sigmoid colon, and rectum. Colorectal cancer is cancer occurring in the colon, and mostly corresponds to adenocarcinoma, and is largely divided into colon cancer and rectal cancer by region.

The colorectal cancer may occur even in any part of the colon/rectum, but about 40% of the colorectal cancer occurs most frequently in the rectum, and next, about 30% thereof occurs in the sigmoid colon, which is near the rectum. Due to changes in dietary habits, even in Korea, the incidence of colorectal cancer and the resultant mortality rate have significantly increased, and the colorectal cancer also act as a major cause of cancer-related deaths in the United States and Europe (American Cancer Society statics for 2009).

Diagnosis of colorectal cancer is simply performed by performing a fecal occult blood test during a medical examination, but actually, additional examinations and tests are needed to confirm colorectal cancer. The tests are mainly performed through colon enema photograph (barium enema photography) and colonoscopy in addition to digital rectal examination and sigmoid colonoscopy, but since the prognosis varies greatly depending on the progression of cancer at the time of diagnosis, early detection of colorectal cancer patients is very important to increase the survival rate of patients.

Meanwhile, epigenetics is a field that studies the regulation of gene expression in a state in which the base sequence of DNA is not changed. The epigenetics is to study the regulation of gene expression through epigenetic variations, such as DNA methylation, and acetylation, methylation, phosphorylation, and ubiquitination of miRNAs or histones, and the like.

Among the epigenetic variations, the DNA methylation is the most studied epigenetic variation. The epigenetic variation may result in gene function variations and changes to tumor cells. Accordingly, the DNA methylation is associated with the expression (or inhibition and induction) of regulatory genes of intracellular diseases, and recently, cancer diagnosis methods through measurement of DNA methylation have been proposed. In particular, since cancer-specific methylation frequently occurs in advance even in precancerous tissues, the detection of cancer-specific methylation is highly likely to be used for diagnosis of cancer.

Therefore, there is a need for development of effective specific methylation markers to colorectal cancer, rectal cancer or colorectal adenoma that can predict the risk of colorectal cancer, and rectal cancer and colorectal adenoma having similar characteristics to the colorectal cancer.

### DISCLOSURE

### TECHNICAL PROBLEM

Therefore, the present inventors found that CpG sites of a specific gene were hypermethylated in colorectal cancer, rectal cancer, or colorectal adenoma, developed a composition, a kit, a nucleic acid chip, and a method capable of diagnosing colorectal cancer, rectal cancer, or colorectal adenoma by detecting the methylation level, and then completed the present invention.

An object of the present invention is to provide a composition for diagnosing colorectal cancer, rectal cancer, or colorectal adenoma comprising a preparation for measuring the methylation level of CpG sites of a specific gene.

An object of the present invention is also to provide a composition for diagnosing colorectal cancer, rectal cancer, or colorectal adenoma consisting of a preparation for measuring the methylation level of CpG sites of a specific gene.

An object of the present invention is also to provide a composition for diagnosing colorectal cancer, rectal cancer, or colorectal adenoma essentially consisting of a preparation for measuring the methylation level of CpG sites of a specific gene.

Another object of the present invention is to provide a kit for diagnosing colorectal cancer, rectal cancer, or colorectal adenoma comprising a PCR primer pair for amplifying fragments including CpG sites of a specific gene and a sequencing primer for pyrosequencing a PCR product amplified by the primer pair.

Yet another object of the present invention is to provide a nucleic acid chip for diagnosing colorectal cancer, rectal cancer, or colorectal adenoma in which probes capable of hybridizing with fragments including CpG sites of a specific gene under a stringent condition are immobilized.

Yet another object of the present invention is to provide a method for providing information for diagnosing colorectal cancer, rectal cancer, or colorectal adenoma comprising measuring the methylation level of CpG sites of a specific gene from a patient's sample and determining the occurrence of colorectal cancer, rectal cancer, or colorectal adenoma based thereon.

Yet another object of the present invention is to provide the use of a preparation for measuring the methylation level of CpG sites of a GLRB gene for preparing a preparation for diagnosing colorectal cancer, rectal cancer, or colorectal adenoma.

Yet another object of the present invention is to provide a method for diagnosing colorectal cancer, rectal cancer, or colorectal adenoma comprising the steps of:
a) obtaining a sample from a subject;
b) measuring the methylation level of CpG sites of a GLRB gene from the sample; and
c) determining the occurrence of colorectal cancer, rectal cancer, or colorectal adenoma based on the measured methylation level.

### TECHNICAL SOLUTION

In order to achieve the object, the present invention provides a composition for diagnosing colorectal cancer, rectal cancer, or colorectal adenoma comprising a preparation for measuring the methylation level of CpG sites of a GLRB gene.

The present invention also provides a composition for diagnosing colorectal cancer, rectal cancer, or colorectal adenoma consisting of a preparation for measuring the methylation level of CpG sites of a GLRB gene.

The present invention also provides a composition for diagnosing colorectal cancer, rectal cancer, or colorectal adenoma essentially consisting of a preparation for measuring the methylation level of CpG sites of a GLRB gene.

In order to achieve another object of the present invention, the present invention provides a kit for diagnosing colorectal cancer, rectal cancer, or colorectal adenoma comprising a primer pair for amplifying fragments including CpG sites of a GLRB gene.

In order to achieve yet another object of the present invention, the present invention provides a nucleic acid chip for diagnosing colorectal cancer, rectal cancer, or colorectal adenoma in which probes capable of hybridizing with fragments including CpG sites of a GLRB gene are immobilized.

In order to achieve yet another object of the present invention, the present invention provides a method for providing information for diagnosing colorectal cancer, rectal cancer, or colorectal adenoma comprising measuring the methylation level of CpG sites of a GLRB gene from a patient's sample suspected of having colorectal cancer, rectal cancer, or colorectal adenoma; and
determining the occurrence of colorectal cancer, rectal cancer, or colorectal adenoma based on the measured methylation level.

In order to achieve yet another object of the present invention, the present invention provides the use of a preparation for measuring the methylation level of CpG sites of a GLRB gene for preparing a preparation for diagnosing colorectal cancer, rectal cancer, or colorectal adenoma.

In order to achieve yet another object of the present invention, the present invention provides a method for diagnosing colorectal cancer, rectal cancer, or colorectal adenoma comprising the steps of:
a) obtaining a sample from a subject;
b) measuring the methylation level of CpG sites of a GLRB gene from the sample; and
c) determining the occurrence of colorectal cancer, rectal cancer, or colorectal adenoma based on the measured methylation level.

Unless otherwise defined, all technical and scientific terms used herein have the same meaning as commonly understood by those skilled in the art. The following references provide one skill with general definitions of various terms used in the present specification: Singleton et al., DICTIONARY OF MICROBIOLOGY AND MOLECULAR BIOLOTY(2th ed. 1994); THE CAMBRIDGE DICTIONARY OF SCIENCE AND TECHNOLOGY(Walkered., 1988); and Hale & Marham, THE HARPER COLLINS DICTIONARY OF BIOLOGY.

Hereinafter, the present invention will be described in detail.

The present invention provides a composition for diagnosing colorectal cancer, rectal cancer, or colorectal adenoma comprising a preparation for measuring the methylation level of CpG sites of a GLRB gene.

As used herein, the term "methylation" means that a methyl group is attached to a base constituting DNA. Preferably, in the present invention, the methylation means whether methylation occurs in cytosine, a specific CpG site of a specific gene. When the methylation occurs, the binding of a transcription factor is disturbed to inhibit the expression of the specific gene. Conversely, when unmethylation or hypomethylation occurs, the expression of the specific gene is increased.

In genomic DNA of mammalian cells, in addition to A, C, G, and T, there is the fifth base called 5-methylcytosine (5-mC) with a methyl group attached to the fifth carbon of a cytosine ring. The methylation of 5-methylcytosine occurs only at C of CG dinucleotide (5'-mCG-3') called CpG, and the methylation of CpG inhibits the expression of alu or transposon and a repeating sequence of the genome. In addition, since the 5-mC of the CpG is naturally deaminated to be easily thymine (T), the CpG is a site where most epigenetic changes frequently occur in mammalian cells.

As used herein, the term "measuring of the methylation level" refers to measuring the methylation level of the CpG sites of the GLRB gene, and may be measured through a detection method according to bisulfite treatment or a bisulfite-free detection method. The methylation level may be measured by methylation-specific PCR, for example, methylation-specific polymerase chain reaction (MSP), real time methylation-specific polymerase chain reaction (PCR), PCR using a methylated DNA-specific binding protein, quantitative PCR, or the like. Alternatively, the methylation level may be measured by automatic sequencing such as pyrosequencing and bisulfite sequencing, but is not limited thereto. In addition, the methylation level may be measured using a ten-eleven translocation (TET) protein as a bisulfite-free detection method. The TET protein is an enzyme that acts on DNA and is involved in chemical changes of bases, and when bisulfite is treated, all Cs except for methylated C are converted to T bases, whereas in the TET protein, only the methylated C is converted to T to enable effective detection.

Preferably, the CpG site of the GLRB gene refers to a CpG site present on DNA of the gene. The DNA of the gene is a concept including all of a series of structural units required for expression of the gene and operably linked to each other, and may include, for example, a promoter region, a protein coding region (open reading frame, ORF) and a terminator region. Accordingly, the CpG sites of the GLRB gene may present in the promoter region, the protein coding region (open reading frame, ORF) or the terminator region of the corresponding gene.

Preferably, in the present invention, the measuring of the methylation level of the CpG sites of the GLRB gene may mean measuring the cytosine methylation level of the CpG sites of the gene shown in Table 1 below.

**Table 1**

| Symbol | Genome Build | Chromosome | Region |
|---|---|---|---|
| GLRB | GRCh37 | 4 | 157997167-157997686 |

In the present invention, the CpG site of the GLRB gene is located to +/- 2000 bases (2 kb) from a transcription start site (TSS) of the gene.

In the present invention, a base sequence of a human genome chromosomal region was expressed according to The February 2009 Human reference sequence (GRCh37), but the expression of a specific sequence of the human genome chromosomal region may be slightly changed as the study results on the genomic sequence are updated, and the expression of the human genome chromosomal region of the present invention may vary according to the change. Accordingly, in the human genome chromosomal region expressed according to The February 2009 Human reference sequence (GRCh 37) of the present invention, since a human reference sequence has been updated after the filing date of the present invention, even if the expression of the human genome chromosomal region is changed differently from now, it will be apparent that the scope of the present invention affects the changed human genome chromosomal region. These changes may be easily seen by those skilled in the art to which the present invention pertains.

In the present invention, a preparation for measuring the methylation level of the CpG sites may include a compound that modifies a cytosine base or a methylation sensitive restriction enzyme, a primer specific to a methylated allele sequence of the GLRB gene, and a primer specific to an unmethylated allele sequence.

The compound that modifies the cytosine base is a compound of modifying unmethylated cytosine or methylated cytosine, and may be bisulfite or a salt thereof, preferably sodium bisulfite of modifying the unmethylated cytosine, or a TET protein of modifying the methylated cytosine, but is not limited thereto. Methods for detecting whether the CpG sites are methylated by modifying the cytosine base are well known in the art (WO01/26536; US2003/0148326A1).

In addition, the methylation-sensitive restriction enzyme may be a restriction enzyme capable of specifically detecting the methylation of the CpG site, and may be a restriction enzyme containing CG as a recognition site of the restriction enzyme. For example, the methylation-sensitive restriction enzyme includes SmaI, SacII, EagI, HpaII, MspI, BssHII, BstUI, NotI, and the like, but is not limited thereto. Depending on methylation or unmethylation at C of the restriction enzyme recognition site, cleavage by the restriction enzyme varies and may be detected through PCR or southern blot analysis. Methylation-sensitive restriction enzymes other than the restriction enzymes are well known in the art.

The primer may include a primer specific to the methylated allele sequence of the GLRB gene and a primer specific to the unmethylated allele sequence.

In the present invention, the term "primer" refers to a nucleic acid sequence having a short free 3-terminal hydroxyl group, and a short nucleic acid sequence capable of forming a base pair with a complementary template and serving as a starting point for copying a template strand. The primer may initiate DNA synthesis in the presence of a reagent for polymerization (i.e., DNA polymerase or reverse transcriptase) and four different nucleoside triphosphates in appropriate buffer and temperature. In addition, the primer is a sense and antisense nucleic acid with a sequence of 7 to 50 nucleotides and may incorporate an additional feature without changing the basic properties of the primer that serves as an initiation site of DNA synthesis.

The primer of the present invention may be preferably designed according to a sequence of a specific CpG site to analyze the methylation, and more preferably, may be at least one selected the group consisting of a primer pair capable of specifically amplifying cytosine that is methylated to be unmodified by bisulfite, a primer pair capable of specifically amplifying cytosine that is not methylated to be modified by bisulfite, and a primer pair capable of specifically amplifying cytosine that is methylated to be modified by a TET-based protein, and a primer pair capable of specifically amplifying cytosine that is not methylated and has not been modified by a TET-based protein.

Accordingly, the present invention provides a kit for diagnosing colorectal cancer, rectal cancer, or colorectal adenoma comprising a primer pair for amplifying fragments including CpG sites of a GLRB gene.

In the composition and the kit, in addition to the preparation, polymerase agarose, a buffer solution required for electrophoresis, and the like may be additionally included.

In addition, the present invention provides a nucleic acid chip for diagnosing colorectal cancer, rectal cancer, or colorectal adenoma in which probes capable of hybridizing with fragments including CpG sites of a GLRB gene are immobilized.

In the present invention, the term "nucleic acid" refers to oligonucleotide, nucleotide, polynucleotide or fragments thereof, single- or double-stranded genomic or synthetic-origin DNA or RNA, sense or antisense-stranded genomic or synthetic-origin DNA or RNA, and peptide nucleic acid (PNA) or natural or synthetic-origin DNA or RNA analog material. If the nucleic acid is RNA, it will be apparent to those skilled in the art that instead of deoxynucleotides A, G, C and T, ribonucleotides A, G, C and U are substituted, respectively.

Since the methylation starts from the outside of a regulatory site of the gene and proceeds to the inside thereof, the gene involved in cell transformation may be diagnosed early by detecting the methylation outside the regulatory site.

Accordingly, it is possible to early diagnose cells likely to form colorectal cancer, rectal cancer, or colorectal adenoma using the methylated gene marker. When a gene confirmed to be methylated in cancer cells is methylated in clinically or morphologically normal-appearing cells, the normal-appearing cells are being cancerizated. Therefore, since the methylation of a colorectal cancer, rectal cancer, or colorectal adenoma-specific gene in normal-appearing cells is confirmed, it is possible to early diagnose colorectal cancer, rectal cancer, or colorectal adenoma.

In addition, the present invention provides a method for providing information for diagnosing colorectal cancer, rectal cancer, or colorectal adenoma comprising measuring the methylation level of CpG sites of a GLRB gene from a patient's sample suspected of having colorectal cancer, rectal cancer, or colorectal adenoma; and
determining the occurrence of colorectal cancer, rectal cancer, or colorectal adenoma based on the measured methylation level.

The method for measuring the methylation level may be selected from the group consisting of PCR, methylation specific PCR, real time methylation specific PCR, PCR using a methylated DNA-specific binding protein, measuring whether there is methylation using a methylation-sensitive restriction enzyme, quantitative PCR, DNA chip, pyrosequencing and bisulfite sequencing, but is not limited thereto.

Specifically, the methylation-specific PCR method is a method of designing and using different types of primers depending on whether CpG dinucleotide is methylated in a primer to perform PCR after treating a sample DNA with bisulfite. If a primer binding site has been methylated, PCR is performed by the methylated primer, and if not methylated, PCR is performed by a normal primer. That is, the methylation-specific PCR method is a method of treating the sample DNA with bisulfite, performing PCR using two types of primers at the same time, and comparing the results.

The real time methylation specific PCR is to convert the methylation-specific PCR method to a real-time measurement method and to perform real time PCR by treating genomic DNA with bisulfite, designing PCR primers corresponding to methylation, and using these primers. At this time, there are two methods: a detection method using a TaqMan probe complementary to an amplified base sequence and a detection method using SYBRgreen. Accordingly, the real time methylation specific PCR may selectively quantitative-analyze only methylated DNA. In this case, the real time methylation specific PCR is a method of preparing a standard curve using an in vitro methylated DNA sample, and quantitatively analyzing the methylation level by amplifying a gene without a 5'-CpG-3' sequence in a base sequence together as a negative control for standization.

In the method for measuring the methylation using the methylation-sensitive restriction enzyme, the methylation-sensitive restriction enzyme uses CpG dinucleotide as an action site, and does not act as the enzyme when this site is methylated. Therefore, when the sample DNA is treated with the methylation-sensitive restriction enzyme and then amplified by PCR so as to include an enzyme target site, in the case of the methylated site, the restriction enzyme does not act, but is amplified by PCR, but the unmethylated normal site is cleaved by the restriction enzyme and not amplified by PCR, thereby measuring the methylation of a specific DNA site.

In the PCR or DNA chip method using the methylated DNA-specific binding protein, when a protein that specifically binds only to methylated DNA is mixed with DNA, the protein specifically binds only to the methylated DNA, so that only the methylated DNA may be selectively isolated. After mixing genomic DNA with the methylated DNA-specific binding protein, only the methylated DNA is selectively isolated. This method is a method of amplifying these isolated DNAs using a PCR primer corresponding to an intron site, and then measuring the methylation by agarose electrophoresis. In addition, the methylation may be measured even by quantitative PCR, and the methylated DNA isolated by the methylated DNA-specific binding protein is labeled with a fluorescent dye and hybridized to a DNA chip integrated with a complementary probe to measure the methylation. Here, the methylated DNA-specific binding protein is not limited to MBD2bt.

In addition, pyrosequencing of bisulfite-treated DNA is based on the following principle. When the methylation occurs at a CpG dinucleotide site, 5-methylcytosine (5-mC) is formed, and in this case, the modified base is changed to uracil upon treatment with bisulfite. If the CpG dinucleotide has been methylated when the DNA extracted from the sample is treated with bisulfite, the CpG dinucleotide is preserved as cytosine and the remaining unmethylated cytosines are changed to uracils. Sequencing of the bisulfite-treated DNA may be preferably performed using a pyrosequencing method. The detailed description of pyrosequencing is known in the prior arts [Ronaghi et al, Science 1998 Jul 17, 281(5375), 363-365; Ronaghi et al,Analytical Biochemistry 1996 Nov 1, 242(1), 84-9; Ronaghi et al. Analytical Biochemistry 2000 Nov 15, 286 (2): 282-288; Nyr, P. Methods Mol Biology 2007, 373, 114].

Meanwhile, by the bisulfite-free detection method using the TET protein, only methylated C may be converted to T using the TET protein to detect the base at the methylated region (see LIU, Yibin, et al., Nature Biotechnology volume 37, pages 424-429 (2019)).

When the methylation occurs at the CpG dinucleotide site so that cytosine is formed as 5-methylcytosine (5-mC), the CpG dinucleotide has been methylated when treated with the ten-eleven translocation (TET) protein to be changed to uracil, and unmethylated cytosines are preserved. The sequencing of TET-treated DNA is not limited only to the pyrosequencing method, but may be performed by using methods such as methylation-sensitive PCR (MSP), microarray, next generation sequencing (NGS), and the like.

Preferably, the method for providing information for diagnosing colorectal cancer, rectal cancer, or colorectal adenoma of the present invention may be performed by a method comprising the steps of a) obtaining a sample from a subject, b) obtaining genomic DNA from the sample, c) treating the obtained genomic DNA with a compound for modifying unmethylated cytosine bases, d) obtaining a PCR product by amplifying the treated DNA by PCR using a primer for pyrosequencing capable of amplifying a promoter of a GLRB gene, and e) measuring the methylation level by pyrosequencing the PCR product using a sequencing primer.

The obtaining of the genomic DNA in step b) may be performed using a phenol/chloroform extraction method, an SDS extraction method, a CTAB isolation method, or a commercially available DNA extraction kit, which is commonly used in the art.

In the present invention, the "determining of the occurrence of colorectal cancer, rectal cancer, or colorectal adenoma based on the measured methylation level" may be determining the occurrence of colorectal cancer, rectal cancer, or colorectal adenoma by measuring the methylation level of CpG sites of the GLRB gene from a patient's sample suspected of having colorectal cancer, rectal cancer, or colorectal adenoma and simultaneously or sequentially measuring the methylation level of CpG sites of the same gene in a normal control sample and then comparing both the methylation levels. In addition, the occurrence of colorectal cancer, rectal cancer, or colorectal adenoma may be determined by measuring the methylation level of the CpG sites of the GLRB gene from the normal control sample in advance to set a threshold value, and then comparing the threshold value thereof with the value of the methylation level measured from the patient's sample. When the methylation level of the CpG sites of the GLRB gene from the normal control sample is measured in advance to set the threshold value, without a need to measure the methylation level of the normal control each time for each diagnosis, it is possible to provide information for diagnosing colorectal cancer, rectal cancer, or colorectal adenoma of the corresponding patient rapidly only by measuring the methylation level of the CpG sites of the GLRB gene from the patient's sample.

In the present invention, the term "sample" refers to a wide range of bodily fluids, including all biological fluids obtained from individuals, bodily fluids, cell lines, tissue cultures, etc., depending on a type of analysis to be performed. Methods for obtaining bodily fluids and tissue biopsies from mammals are generally widely known, and in the present invention, the sample may be preferably selected from the group consisting of human derivatives including tissues, cells, blood, plasma, serum, feces, and urine. Since abnormal methylation changes in tumor tissue show significant similarities to methylation changes in genomic DNA obtained from a biological sample such as cells, whole blood, serum, plasma, saliva, sputum, cerebrospinal fluid, or urine, in the case of using the marker of the present invention, with respect to the prediction of the occurrence of colorectal cancer, rectal cancer, or colorectal adenoma, there is an advantage that it is possible to be easily diagnosed through blood, bodily fluids, or the like.

The present invention provides the use of a preparation for measuring the methylation level of CpG sites of a GLRB gene for preparing a preparation for diagnosing colorectal cancer, rectal cancer, or colorectal adenoma.

The present invention provides a method for diagnosing colorectal cancer, rectal cancer, or colorectal adenoma comprising the steps of:
a) obtaining a sample from a subject;
b) measuring the methylation level of CpG sites of a GLRB gene from the sample; and
c) determining the occurrence of colorectal cancer, rectal cancer, or colorectal adenoma based on the measured methylation level.

In an aspect, the present invention provides a method for diagnosing and treating colorectal cancer, rectal cancer, or colorectal adenoma of a subject comprising the following steps:
i) obtaining a sample from a subject;
ii) measuring the methylation level of CpG sites of a GLRB gene from the sample;
iii) determining the occurrence of colorectal cancer, rectal cancer, or colorectal adenoma based on the measured methylation level; and
iv) treating colorectal cancer, rectal cancer, or colorectal adenoma by administering a therapeutic drug for treating bladder cancer to the determined subject or through surgery.

Methods including steps i) to iv) are understood based on the method including steps a) to c) described above.

Step iv) is a step of performing the treatment of the diseases by a means such as administration of the therapeutic drug to the subject in which the disease is diagnosed in step iii) or surgery.

The term 'treatment' of the present invention comprehensively refers to improving colorectal cancer, rectal cancer, or colorectal adenoma, or symptoms thereof, and may include treating or substantially preventing these diseases, or improving the conditions thereof and includes alleviating, treating or preventing a symptom or most of symptoms derived from colorectal cancer, rectal cancer, or colorectal adenoma, but is not limited thereto.

The type of `therapeutic drug' is not particularly limited as long as the therapeutic drug is any type of drug typically used for the treatment of colorectal cancer, rectal cancer, or colorectal adenoma. In addition, the therapeutic drug is administered to a subject in a `therapeutically effective dose', and the therapeutically effective dose for patients may be determined by those skilled in the art by considering various factors, such as the age, weight, health condition, and sex of a patient, the severity of a disease, a diet and an excretion rate as well as unique properties, a route of administration, and a treatment number of the drug. The route of administration of the therapeutic drug is not particularly limited, and the therapeutic drug may be administered orally or parenterally, and the route of administration includes both local administration and systemic administration. The parenteral administration is not limited thereto, but may be, for example, intranasal drug application, subcutaneous injection, and the like, and as another example, a method such as intramuscular injection, intravenous injection, or the like may be used.

The 'sample' of the present invention is isolated and obtained from a subject suspected of having the disease, but is not limited thereto, but may be selected from the group consisting of cells, tissues, blood, serum, plasma, saliva, sputum, mucosal fluid, and urine. The 'subject' may be animals, preferably animals including mammals, particularly humans, and may be cells, tissues, organs, etc. derived from animals. The subject may be a patient requiring the therapeutic effect.

The term "comprising" used herein is used in the same meaning as "including" or "characterized by", and does not exclude additional ingredients or steps of the method which are not specifically mentioned in the composition or the method according to the present invention. The term "consisting of" means excluding additional elements, steps or ingredients, etc., unless otherwise described. The term "essentially consisting of" means including materials or steps which do not substantially affect basic properties thereof in addition to the described materials or steps within the range of the composition or the method.

### ADVANTAGEOUS EFFECTS

As described above, since the hypermethylation of the CpG sites of the GLRB gene is specifically shown in colorectal cancer, rectal cancer, or colorectal adenoma, it is possible to not only accurately and rapidly, but also early diagnose colorectal cancer, rectal cancer, or colorectal adenoma using the composition, the kit, the chip or the method according to the present invention.

### DESCRIPTION OF DRAWINGS

FIG. 1 illustrates a result of confirming methylation information of a GLRB gene in a total of 32 cancer types.
FIG. 2 illustrates a result of confirming the accuracy of diagnosing colorectal cancer of the GLRB gene selected according to the present invention.

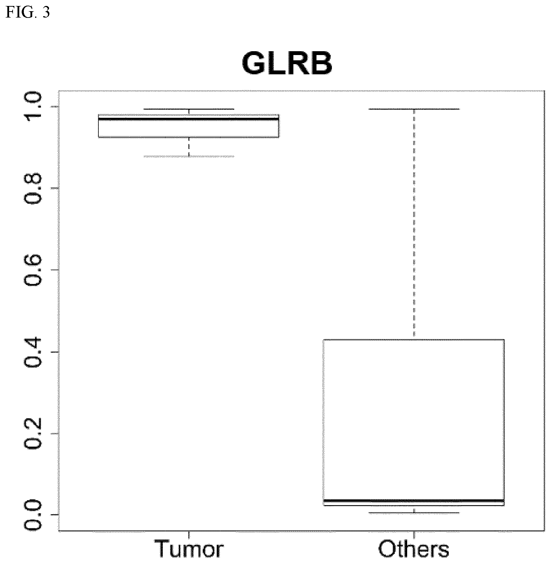

FIG. 3 illustrates a result of confirming a difference in methylation between a colorectal cancer tumor tissue (tumor) cell line group and a non-colorectal cancer tumor tissue (others) cell line group.
FIG. 4 illustrates a result of confirming a difference in methylation between tumor tissue (cancer), colorectal adenoma tissue (adenoma), and normal tissue (normal) in colorectal adenoma.
FIG. 5 illustrates a result of confirming a difference in qMSP-based methylation between tumor tissue (cancer) and tumor adjacent normal tissue (non tumor) in colorectal adenoma.
FIG. 6 illustrates a result of confirming methylation information of an OPLAH gene as Comparative Example.
FIG. 7 illustrates a result of confirming a difference in methylation based on droplet digital PCR (ddPCR) in colorectal adenoma (Adenoma), tumor tissue (Tumor), and tumor adjacent normal tissue (Non-tumor).
FIG. 8 illustrates a result of analyzing a difference in methylation using droplet digital PCR (ddPCR) in serums of a normal person and a colorectal cancer patient.

### MODES FOR THE INVENTION

Hereinafter, preferred Examples will be proposed in order to help in understanding of the present invention. However, the following Examples are just provided to more easily understand the present invention and the contents of the present invention are not limited by Examples.

### Example 1: Screening of colorectal cancer-specific methylated genes

To screen methylated genes specifically found in colorectal cancer, a large-scale comparative study on methylation between tumor tissue obtained from cancer surgery of patients with colorectal cancer and normal tissue was conducted using data on two large-scale methylation microarray chips (see Table 2). The tumor tissue used in the corresponding study means tumor tissue of colorectal cancer, and the non-tumor tissue means tissues other than the tumor tissue including normal tissue.

**Table 2**

| | dataset#1 | dataset#2 | Total |
|---|---|---|---|
| Tumor | 112 | 395 | 507 |
| Non-tumor | 149 | 45 | 194 |

In order to screen colorectal cancer-specific methylated genes, DNA was extracted from each tissue, and the methylation level of a genetic region was confirmed using an Infinium Human Methylation 450 Beadchip microarray.

The DNA extracted from each tissue was converted through bisulfite treatment. Through this, a cytosine base was modified depending on the methylation of the DNA region. A probe used in the corresponding microarray experiment was designed specifically to methylation and unmethylation in order to confirm whether a cytosine base in a methylated region of the gene was modified.

The microarray experiment measured the methylation level of the gene through about 450,000 (450 k) probes representing the methylated region of each gene, and a result of each probe derived from the experiment was presented as a beta value. The beta value had a value of 0 to 1, and it was determined that the closer to 1, the higher the methylation level of the corresponding genetic region.

In order to identify differentially methylated regions (DMRs) between a tumor group and a non-tumor group, by using an empirical Bayes t-test, a Linear Models for Microarray Data (Limma) method, a genetic region having a statistically significant difference in methylation between the groups was identified.

The Limma method has been known to be least affected by an outlier among several methylation statistical analysis methods to identify the difference between the groups. Therefore, the method was a suitable method for finding cancer-specific markers because the method was less affected by abnormal measurement values of some samples. In the present experiment, it was determined that there was a significant difference in methylation between the two groups as an adjusted p-value derived through the Limma method was smaller.

In particular, in order to search for tumor-specific methylated regions, among genetic regions with a significant difference in beta value between the tumor and non-tumor groups, regions with higher methylation in tumor tissue than in non-tumor tissue were selected as cancer-specific biomarker candidates.

As a result, as a result of Limma analysis in each of the two datasets, compared with the non-tumor group, when comparing the tumor groups, genetic regions having a significantly low p value (top 10% with lowest P value) and a large difference in beta value of 0.2 or more between the groups were selected as tumor-specific hypermethylated regions. As a result, among about 450,000 genetic regions, 3,878 genetic regions showing tumor-specific hypermethylation commonly in both the datasets were selected as biomarker candidates.

### Example 2: Screening of colorectal cancer-specific hypermethylated genes

In the 3,878 genetic regions as the biomarkers identified in Example 1, the methylation level of each corresponding region in tumors other than colorectal cancer was identified and compared to find a colorectal cancer or colorectal adenoma-specific genetic region among the biomarkers. As a result of analyzing the DNA methylation 450k array test results of The Cancer Genome Atlas (TCGA), a public cancer gene database, methylation information of genetic regions corresponding to 32 cancer types was confirmed. Among these, as a result of confirming genetic regions with significantly higher beta values in colorectal cancer, rectal cancer, or colorectal adenoma compared to other 30 types of cancer other than colorectal cancer and rectal cancer, it was confirmed that the genetic region of the GLRB gene among the 3,878 genetic regions caused specific methylation to colorectal cancer, rectal cancer, or colorectal adenoma.

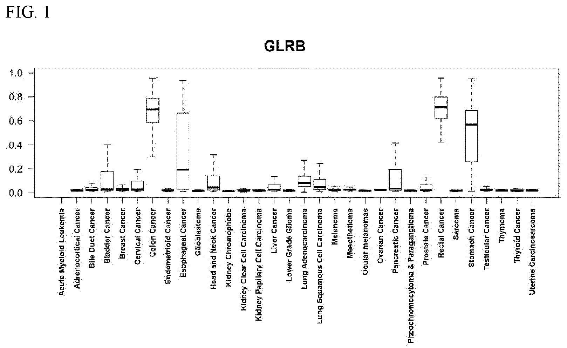

The methylation level of the gene was as shown in FIG. 1 through the microarray experiment on tumor tissue (tumor tissue of colorectal cancer) and non-tumor tissue (tissue other than tumor tissue including normal tissue) for the gene. In the methylation level, the result of each probe derived through the experiment was represented as a beta value, and the beta value had a value of 0 to 1, and it was determined that the closer to 1, the higher the methylation level of the corresponding genetic region.

Meanwhile, in the case of the genetic region when the methylation difference was observed when comparing tumor tissue of colorectal cancer, rectal cancer, or colorectal adenoma and non-tumor tissue, methylation may also occur even with respect to cancers other than colorectal cancer, rectal cancer, or colorectal adenoma. That is, colorectal cancer, rectal cancer, or colorectal adenoma-specific methylation was not confirmed.

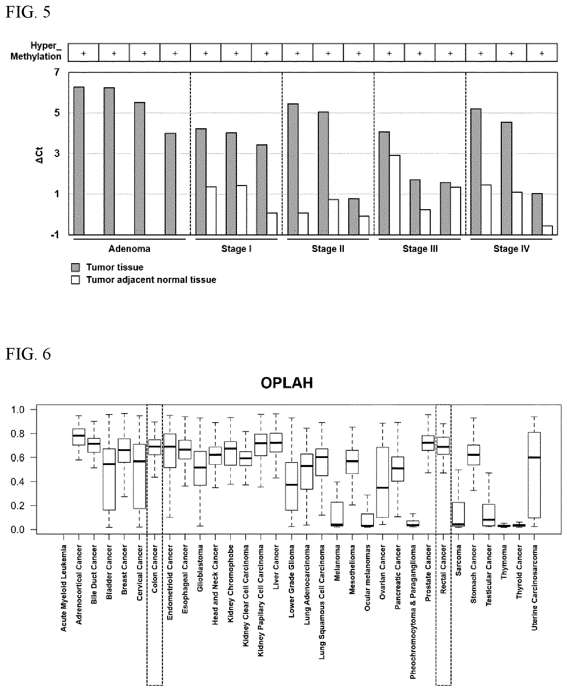

For example, in the case of a 5-oxoprolinase (OPLAH, ATP-hydrolysing) gene, among the 3,878 genetic regions identified in Example 1, there was one having the largest difference in methylation between tumor tissue and non-tumor tissue, but as shown in FIG. 5, it was confirmed that high methylation occurred in all cancer types except for acute myeloid leukemia, occular melanomas, pheochromocytoma&paraganglioma, thymoma, thyroid cancer, and the like.

The 32 cancer types were as follows: Acute Myeloid Leukemia, Adrenocortical cancer, Bile Duct cancer, Breast cancer, Cervical Cancer, Colon cancer, Endometrioid Cancer, Esophageal Cancer, Glioblastoma, Head and neck cancer, Kidney chromophobe, Kidney Clear cell carcinoma, Kidney papillary cell carcinoma, Large b-cell lymphoma, Liver cancer, Lower Grade Glioma, Lung adenocarcinoma, Melanoma, Mesothelioma, Ocular melanomas, Ovarian cancer, Pancreatic cancer, Pheochromocytoma&paraganglioma, Prostate cancer, Rectal cancer, Sarcoma, Stomach cancer, Testicular cancer, Thymoma, Thyroid cancer, and Uterine carcinosarcoma.

Among these genetic regions, when the corresponding region was not a pseudogene, existed in a CpG island site, was located in a genetic region selected to +/-2000 bases (2 kb) from a transcription start site (TSS) of the gene, and existed in an autosome, the corresponding region was selected as a colorectal cancer-specific hypermethylated gene. As a result, as shown in Table 3 below, one gene was selected (see FIG. 1).

**Table 3**

| Symbol | Name | Location (Chromosome) | CpG Island |
|---|---|---|---|
| GLRB | glycine receptor beta | 4 | Island |

### Example 3: Confirmation of colorectal cancer specificity of selected genes in cell lines

In order to confirm whether the selected GLRB gene exhibited methylation specific to colorectal cancer or rectal cancer distinguished from other cancers, methylation patterns in 1,022 cancer cell lines derived from largely 14 tissues were analyzed using a public database. For the corresponding data, the Infinium Human Methylation 450 Beadchip microarray experiment was performed on DNA extracted from each cell line according to a manufacturer's standardized methylation analysis test procedure.

In the result values of the performed experiment, the methylation level of the gene was measured through about 450,000 probes as in Example 1, and the methylation value of each probe was presented as a beta value. The beta value had a value of 0 to 1, and it was determined that the closer to 1, the higher the methylation level of the corresponding genetic region.

The 14 tissues were as follows: aerodigestive tract, blood, bone, breast, digestive system, kidney, lung, nervous system, pancreas, skin, soft tissue, thyroid, urogenital system, and other tissue.

In order to confirm the colorectal cancer, rectal cancer, or colorectal adenoma-specific methylation of the selected GLRB gene, methylation data derived from 1,022 cell lines were largely classified into a colorectal cancer cell line group (n = 51) and a non-colorectal cancer cell line group (n = 971).

In order to identify differentially methylated regions (DMRs) between the two classified groups, by using an empirical Bayes t-test, a Linear Models for Microarray Data (Limma) method, genetic regions having a statistically significant difference in methylation between the groups were identified.

**Table 4**

| Difference in methylation between colorectal cancer cell line group and non-colorectal cancer cell line group of selected GLRB gene | | |
|---|---|---|
| **Symbol** | **Difference (average Δ**β**)** | **adjusted p-value** |
| GLRB | 0.65 | 7.05e-31 |

Even in analysis using the cell lines, it was confirmed that the GLRB gene had a significantly lower adjusted p-value even in colorectal cancer and rectal cancer cell lines compared to other cancer cell lines to be colorectal cancer-specific.

### Example 4: Evaluation of diagnostic performance of diagnostic marker candidates of colorectal cancer, rectal cancer, or colorectal adenoma

In order to confirm the usefulness of the selected gene as diagnostic markers in colorectal cancer, the accuracy of diagnosis for colorectal cancer according to a methylation level was evaluated.

In order to evaluate the accuracy of diagnosis, sensitivity and specificity were used. A receiver operating characteristic (ROC) curve representing changes in sensitivity and specificity according to a cut-off value may be shown through the calculation of the sensitivity and specificity values for feasible cut-off values of consecutive diagnostic test measurement values. The accuracy of diagnosis may be measured by an area under the ROC curve (AUC). The AUC value has a value between 0.5 and 1, and it is evaluated that the higher the value, the higher the diagnostic accuracy. If the AUC value is 1, it is meant that the diagnostic result is a perfectly accurate test, but if the AUC value is 0.5, it is determined that the diagnostic result is the same as the random result.

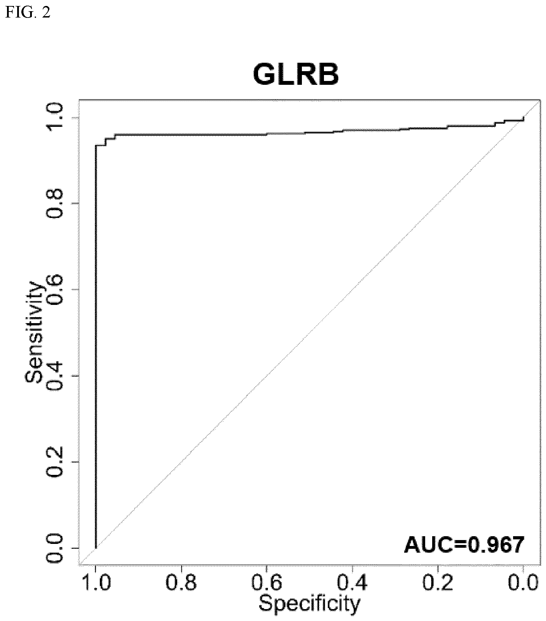

As a result of analyzing the accuracy of cancer classification according to the methylation level between the non-tumor tissue and the tumor tissue using the selected genes by using a collected methylation dataset, as illustrated in FIG. 2, it was confirmed that all the selected genes had Area Under Curve (AUC) values of 0.920 or higher to have high diagnosis accuracy, so that the selected genes were useful for diagnosing colorectal cancer.

### Example 5: Confirmation of methylation in adenoma of selected gene

Adenoma is a disease at a stage before progressing to colorectal cancer, and most colorectal cancers occurs from adenoma. Therefore, rapid detection of adenoma is required for early diagnosis of colorectal cancer. In order to confirm whether the hypermethylation biomarkers selected through previous studies show the characteristics of hypermethylation even in adenoma, the hypermethylation characteristics of genes selected from 64 tumor tissues of colorectal cancer, 42 colorectal adenoma tissues, and 41 non-tumor tissues were examined.

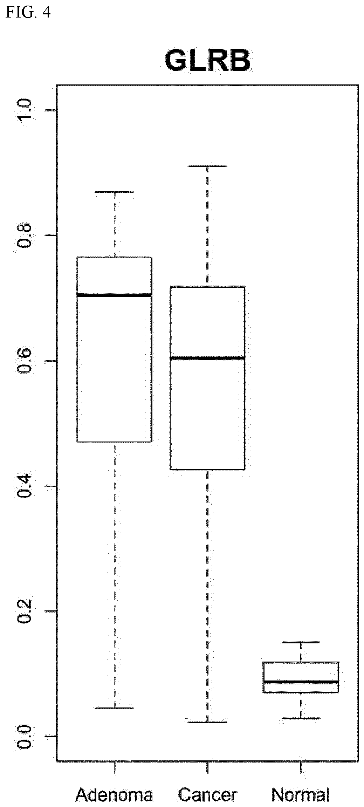

As a result of analyzing the methylation data derived from the Human Methylation450 Beadchip microarray experiment, as shown in FIG. 4, it was confirmed that the selected genes exhibited the same significant hypermethylation characteristic pattern as the non-tumor tissue not only in colorectal cancer but also in colorectal adenoma.

As such a result, it was found that the selected genes may be used even for the diagnosis colorectal adenoma as well as colorectal cancer.

### Example 6: Measurement of qMSP-based methylation in tissue of selected gene

In order to confirm colorectal cancer and adenoma-specific methylation of the GLRB gene in tumor tissue, a difference in methylation between tumor tissue and non-tumor tissue was measured using a quantitative methylation specific PCR (qMSP) technique. To this end, genomic DNA was isolated from a pair of tumor tissue and tumor adjacent tissue of 12 colorectal cancer patients and tumor tissue of 4 adenoma patients, and treated with bisulfite, and then the amplification and methylation level of a GLRB-specific genetic region were observed according to a generalized qMSP experimental method.

In addition, an ACTB gene, which was specifically bound to a genetic region modified by bisulfite to be amplified and not related to methylation to standardize the amplified value of the corresponding region, was used.

The methylation level of DNA modified by bisulfite amplified by PCR was represented as ΔCt, which was a value adjusted with a cycle of threshold (Ct) value of ACTB used as an internal control. ΔCt is defined as follows.

ΔCt = Ct value of ACTB gene - Ct value of gene to be detected

As shown in FIG. 5, it was confirmed that the methylation of the GLRB gene had a relatively high ΔCt value regardless of a tumor stage in colorectal cancer tissue compared to tumor adjacent normal tissue, and particularly, had a very high ΔCt value in adenoma as a precancerous stage, so that the GLRB gene was hypermethylated in colorectal cancer and colorectal adenoma. This is a result that actually shows that the methylation of the selected GLRB gene is effective as a biomarker for the diagnosis of colorectal cancer, particularly early diagnosis.

As such a result, it was found that the selected gene may be used even for the diagnosis of colorectal adenoma as well as colorectal cancer.

### Example 7: Measurement of digital PCR-based methylation in tissue of selected gene

A difference in methylation between tumor tissue and non-tumor tissue was measured using a digital PCR technique that showed excellent detection sensitivity compared to a general real-time PCR method. To this end, genomic DNA was isolated from pairs (4 pairs by tumor stage) of tumor tissue and tumor adjacent tissue of 16 colorectal cancer patients and tumor tissue of 5 adenoma patients, and treated with bisulfite, and then the amplification and methylation level of a GLRB-specific genetic region were observed according to a droplet digital PCR (ddPCR) method.

An ACTB gene was used as an internal control for the modification of the genetic region by bisulfite and the amplification of the genetic region using PCR.

The methylation level of amplifying cfDNA modified by bisulfite by ddPCR was calculated as the copy number.

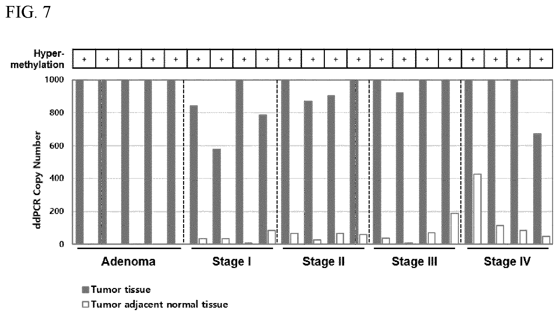

As shown in FIG. 7, it was confirmed that the methylation of the GLRB gene had a relatively high copy number regardless of a tumor stage in colorectal cancer tissue compared to tumor adjacent normal tissue, and particularly, had a very high copy number in adenoma as a precancerous stage, so that the GLRB gene was hypermethylated in colorectal cancer and colorectal adenoma like the result using Real-time PCR. This is a result of showing that the methylation of the selected GLRB gene can be effectively detected not only by real-time PCR but also by digital PCR.

As such a result, it was found that the selected gene can be diagnosed using various molecular biological techniques.

### Example 8: Measurement of digital PCR-Based methylation in plasma

In order to confirm the specific methylation of colorectal cancer in a blood sample, a difference in methylation between colorectal cancer patients and normal persons was measured using ddPCR. To this end, cell-free DNA (cfDNA) was isolated by collecting plasma samples from 10 colorectal cancer patients and plasma samples from normal persons and treated with bisulfite, and then the amplification and methylation level of the GLRB-specific genetic region were observed according to a ddPCR test method.

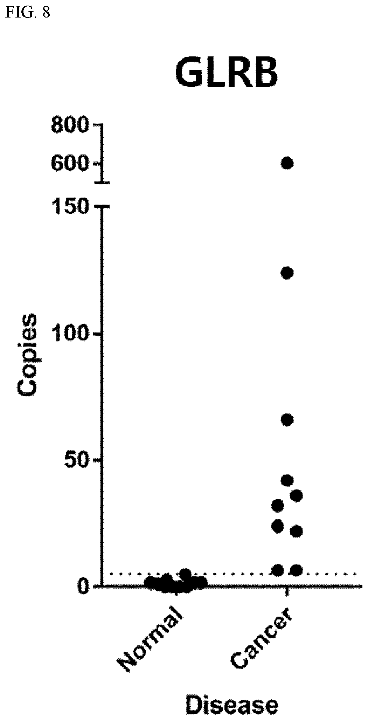

As shown in FIG. 8, it was shown that the methylation of the GLRB gene showed a high methylation level in colorectal cancer plasma samples compared to normal persons, and may be measured with 100% accuracy. This is a result of showing that the methylation of the selected GLRB gene is effective for the diagnosis of not only tissue-but also blood-based colorectal cancer.

### INDUSTRIAL APPLICABILITY

As described above, since the hypermethylation of the CpG sites of the GLRB gene is specifically shown in colorectal cancer, rectal cancer, or colorectal adenoma, it is possible to not only accurately and rapidly but also early diagnose colorectal cancer, rectal cancer, or colorectal adenoma using the composition, the kit, the chip or the method according to the present invention.

## Claims

1. A composition for diagnosing colon cancer, rectal cancer, or colorectal adenoma comprising a preparation for measuring the methylation level of CpG sites of a GLRB gene.

2. The composition of claim 1, wherein the CpG site is located at between a transcription start site of the gene and +/- 2000 bases therefrom.

3. The composition of claim 1, wherein the preparation for measuring the methylation level of the gene CpG site is selected from the group consisting of a compound that modifies an unmethylated cytosine or methylated cytosine base;
a primer specific to a methylated sequence of the CpG sites of the GLRB gene; and
a primer specific to an unmethylated sequence.

4. The composition of claim 3, wherein the compound of modifying the unmethylated cytosine base is bisulfite or a salt thereof and the compound of modifying the methylated cytosine base is a TET protein.

5. The composition of claim 1, wherein the method for measuring the methylation level is selected from the group consisting of a bisulfite-free detection method, methylation-specific polymerase chain reaction, real time methylation-specific polymerase chain reaction, PCR using a methylated DNA-specific binding protein or methylation sensitive restriction enzyme, quantitative PCR, pyrosequencing, and bisulfite sequencing, and Next Generation Sequencing (NGS).

6. A kit for diagnosing colon cancer, rectal cancer, or colorectal adenoma comprising a primer pair for amplifying fragments including CpG sites of a GLRB gene.

7. A nucleic acid chip for diagnosing colon cancer, rectal cancer, or colorectal adenoma in which probes capable of hybridizing with fragments including CpG sites of a GLRB gene are immobilized.

8. A method for providing information for diagnosing colon cancer, rectal cancer, or colorectal adenoma comprising:
measuring the methylation level of CpG sites of a GLRB gene from a patient's sample suspected of having colorectal cancer, rectal cancer, or colorectal adenoma; and
determining the occurrence of colorectal cancer, rectal cancer, or colorectal adenoma based on the measured methylation level.

9. The method of claim 7, wherein the method for measuring the methylation level is selected from the group consisting of a bisulfite-free detection method, methylation-specific polymerase chain reaction, real time methylation-specific polymerase chain reaction, PCR using a methylated DNA-specific binding protein or methylation sensitive restriction enzyme, quantitative PCR, pyrosequencing, and bisulfite sequencing, and Next Generation Sequencing (NGS).

10. The method of claim 7, wherein the sample is selected from the group consisting of tissues, cells, blood, plasma, serum, feces, and urine.

11. Use of a preparation for measuring the methylation level of CpG sites of a GLRB gene for preparing an agent for diagnosing colorectal cancer, rectal cancer, or colorectal adenoma.

12. A method for diagnosing colon cancer, rectal cancer, or colorectal adenoma comprising the steps of:
a) obtaining a sample from a subject;
b) measuring the methylation level of CpG sites of a GLRB gene from the sample; and
c) determining the occurrence of colorectal cancer, rectal cancer, or colorectal adenoma based on the measured methylation level.
